# EUROPEAN PATENT APPLICATION

(11) **EP 1 849 877 A1**
(43) Date of publication of application: **31.10.2007**
(21) Application number: 06075943.8
(22) Date of filing: 24.04.2006
(51) Int. Cl.: C12Q 1/68

(54) **Replicative history of T-and B-cell subsets**

(71) Applicant: Erasmus University Medical Center Rotterdam, 3015 GE Rotterdam (NL)
(72) Inventor: van Dongen, Jacobus Johannes Maria, 2914 LE Nieuwekerk aan den IJssel (NL)
(74) Representative: van Loon, C.J.J.

(57) **Abstract**

The invention relates to the field of immunology and immunodiagnostics. More specifically, the invention relates to a method for determining the replicative history of specific subsets of T lymphocytes or B lymphocytes. A method of the invention is among others advantageously used to assess expansion of normal or diseased T-cells or B-cells. Provided is a method for determining the replicative history of a T-cell subset or a B-cell subset, comprising detecting a signal joint nucleotide sequence on a TREC or on a BREC in at least one T-cell or one B-cell of said subset; and detecting a coding joint nucleotide sequence that is formed upon deletion of said TREC or BREC from a chromosome during a TCR or Ig rearrangement in said T-cell or said B-cell, wherein said TCR or Ig gene rearrangement is the last TCR or Ig gene rearrangement before a functional TCR or Ig molecule is formed and wherein the chromosomal coding joint nucleotide sequence is stably retained in the chromosome and preferably involved in the expressed TCR or Ig molecule. Also provided are oligonucleotides and control cells for use in a method of the invention and diagnostic kits comprising oligonucleotides and/or control cells.

### Annex to the application documents - subsequently filed sequences listing

SEQUENCE LISTING

<110>  Erasmus University Medical Center Rotterdam

<120>  Replicative history of T- and B-cell subsets

<130>  P76983EP00

<140>  EP 06075943.8
<141>  2006-04-24

<160>  26

<170>  PatentIn version 3.3

<210>  1
<211>  26
<212>  DNA
<213>  Artificial

<220>
<223>  Primer V gamma 9 RQ-F-1 (set 1)

<400>  1
cattcacaat gtagagaaac aggaca                                          26

<210>  2
<211>  26
<212>  DNA
<213>  Artificial

<220>
<223>  Primer J gamma 1.2 RQ-R-1 (set 1)

<400>  2
aagaaaactt acctgtaatg ataagc                                          26

<210>  3
<211>  26
<212>  DNA
<213>  Artificial

<220>
<223>  Probe Tr V gamma 9 - J gamma 1.2 pan-1 (set 1)

<400>  3
ttgttccggg accaaatacc ttgatt                                          26

<210>  4
<211>  24
<212>  DNA
<213>  Artificial

<220>
<223>  Primer V gamma 9 RQ-F-2 (set 2)

<400>  4
ggtggatagg atacctgaaa cgtc                                            24

<210>  5
<211>  22
<212>  DNA
<213>  Artificial

<220>
<223>  Primer J gamma 1.2 RQ-R-2 (set 2)

<400>  5
cagcctccca tcccttcttt ac                                                22


<210>  6
<211>  30
<212>  DNA
<213>  Artificial

<220>
<223>  Probe T V gamma 9 - J gamma 1.2 pan-2 (set 2)

<400>  6
tggtcccgga acaaagctta tcattacagg                                        30


<210>  7
<211>  23
<212>  DNA
<213>  Artificial

<220>
<223>  Probe  Tr V gamma 9 - J gamma 1.2 can (set 1 and 2)

<400>  7
tgcccaactc ttgcacctcc cac                                               23


<210>  8
<211>  21
<212>  DNA
<213>  Artificial

<220>
<223>  Primer V gamma 9 TREC-F-1 (set 1)

<400>  8
ctgtggcttc ccaattcgta a                                                 21


<210>  9
<211>  19
<212>  DNA
<213>  Artificial

<220>
<223>  Primer J gamma 1.2 TREC-R-1 (set 1)

<400>  9
cgtcagcagg gccaatttt                                                    19

<210>  10
<211>  32
<212>  DNA
<213>  Artificial

<220>
<223>  Probe T V gamma 9 - J gamma 1.2 TREC-1 (set 1)

<400>  10
ttggcacaat tttaagacaa acacaaaggg ag                                    32


<210>  11
<211>  21
<212>  DNA
<213>  Artificial

<220>
<223>  Primer V gamma 9 TREC-F-2 (set 2)

<400>  11
tgaatgggat ggctcaaact g                                                21


<210>  12
<211>  27
<212>  DNA
<213>  Artificial

<220>
<223>  Primer J gamma 1.2 TREC-R-2 (set 2)

<400>  12
agggccaatt tttatgtatt tatgtaa                                          27


<210>  13
<211>  36
<212>  DNA
<213>  Artificial

<220>
<223>  Probe Tr V gamma 9 - J gamma 1.2 TREC-2 (set 2)

<400>  13
agaagccttt ataagaatct ccctttgtgt ttgtct                                36


<210>  14
<211>  20
<212>  DNA
<213>  Artificial

<220>
<223>  Primer V alpha 10 RQ-F-1 (set 1)

<400>  14
atcacagcct cccagctcag                                                  20

<210>  15
<211>  25
<212>  DNA
<213>  Artificial

<220>
<223>  Primer J alpha 18 RQ-R-1 (set 1)

<400>  15
attttcctgg aatagaaagc gactc                                          25


<210>  16
<211>  29
<212>  DNA
<213>  Artificial

<220>
<223>  Probe T V alpha 10 - J alpha 18  pan-1 (set 1)

<400>  16
aggaactcag ttgactgtct ggcctggtg                                      29


<210>  17
<211>  25
<212>  DNA
<213>  Artificial

<220>
<223>  Primer V alpha 10 RQ-F-2 (set 2)

<400>  17
cggaagatat acagcaactc tggat                                          25


<210>  18
<211>  24
<212>  DNA
<213>  Artificial

<220>
<223>  Primer J alpha 18 RQ-R-2 (set 2)

<400>  18
tggaatagaa agcgactcac tcac                                           24


<210>  19
<211>  34
<212>  DNA
<213>  Artificial

<220>
<223>  Probe Tr V alpha 10- J alpha 18 pan-2 (set 2)

<400>  19
gccagacagt caactgagtt cctcttccaa agta                                34


<210>  20

<211>  26
<212>  DNA
<213>  Artificial

<220>
<223>  Probe Tr V alpha 10 - J alpha 18 inv (set 1 and 2)

<400>  20
agcctctgtc gctcaccaca cagatg                                          26


<210>  21
<211>  22
<212>  DNA
<213>  Artificial

<220>
<223>  Primer V alpha 10 TREC-R-1 (set 1)

<400>  21
ccttgataac ctccaggtgc ag                                              22


<210>  22
<211>  21
<212>  DNA
<213>  Artificial

<220>
<223>  Primer J alpha 18 TREC-F-1 (set 1)

<400>  22
gcttaaggag gatggaggca a                                               21


<210>  23
<211>  28
<212>  DNA
<213>  Artificial

<220>
<223>  Probe T V alpha 10 - J alpha 18 TREC-1 (set 1)

<400>  23
atgggaaaca ttagggctgg gttcatgt                                        28


<210>  24
<211>  30
<212>  DNA
<213>  Artificial

<220>
<223>  Primer V alpha 10 TREC-R-2 (set 2)

<400>  24
ctgcataaaa aataacggtc tactctaact                                      30


<210>  25
<211>  22

<212>  DNA
<213>  Artificial

<220>
<223>  Primer J alpha 18 TREC-F-2 (set 2)

<400>  25
aggatggagg caaattttca ac                                                22


<210>  26
<211>  25
<212>  DNA
<213>  Artificial

<220>
<223>  Probe Tr V alpha 10 - J alpha 18 TREC-2 (set 2)

<400>  26
gtttcccatc ccctctcccc tcagc                                             25

## Description

The invention relates to the field of immunology and immunodiagnostics. More specifically, the invention relates to a method for determining the replicative history of lymphocytes. A method of the invention is, among others, advantageously used to assess the extent of expansion of normal or diseased T- or B-cells.

During the first 2 to 3 years of life the immune system encounters many different antigens, which induce expansion and selection of the antigen-specific immune system, i.e. the antigen-receptor expressing B-lymphocytes and T-lymphocytes. This is associated with an increase in the absolute numbers of blood B-lymphocytes and T-lymphocytes from median values of 0.5 x 10⁶/L and 2.5 x 10⁶/L in neonatal cord blood to 1.4 x 10⁶/L and 4.6 x 10⁶/L in the 2^{nd} year of life, respectively.¹ It can be anticipated that the expansion of the B-lymphocytes and T-lymphocytes is at least in part antigen-driven. Consequently, specific B-cell and T-cell subsets are positively selected and expanded based on the reactivity of their antigen-specific receptors, i.e. the immunoglobulin (Ig) and T-cell receptor (TCR) molecules.²

### TCR molecules and TCR genes rearrangements

Two main types of TCR molecules exist, i.e. the classical TCR, consisting of a TCRα and a TCRβ chain. The vast majority of peripheral T-lymphocytes (85-95%) express TCRαβ molecules, and a minority of peripheral T-lymphocytes (5-15%) express TCRγδ molecules. Each TCR chain consists of an antigen-recognizing variable domain and a constant domain. The antigen-recognizing variable domains differ per T-cell and are encoded by different combinations of variable (V), diversity (D), and joining (J) gene segments in case of TCRβ and TCRδ chains and by different combinations of V and J gene segments in case of TCRα and TCRγ chains.²

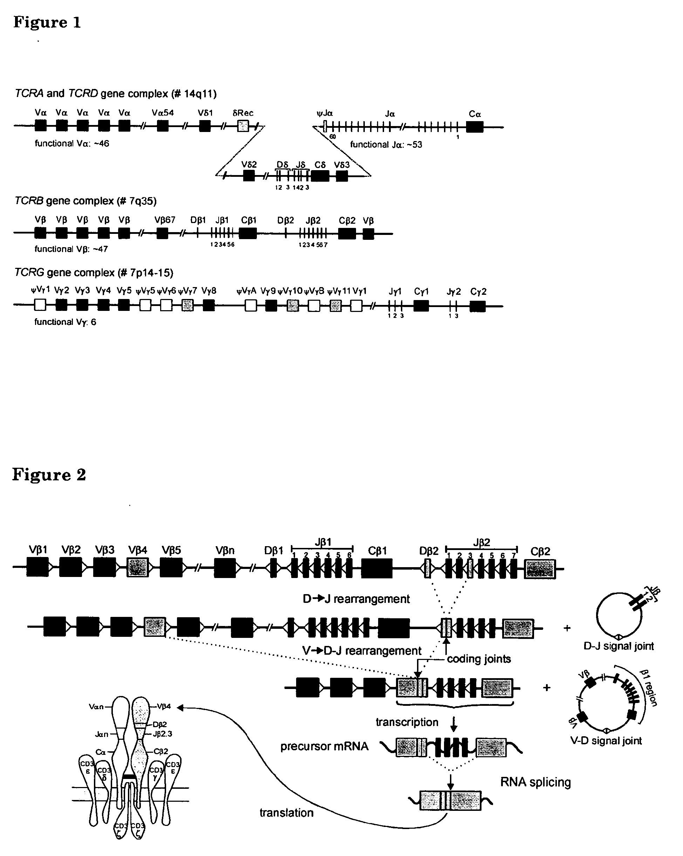

The many different functional Vβ (n≈47), Dβ (n=2), and Jβ (n=13) gene segments in the *TCRB* gene and the many different functional Vα (n≈46) and Jα (n=53) gene segments in the *TCRA* gene determine the potential V(D)J combinatorial repertoire of the TCRαβ molecules, which is estimated to be > 2 x 10⁶ different combinations (Figure 1).²⁻⁴ The combinatorial repertoire of TCRγδ molecules is limited, because of the limited number of functional gene segments: 6 Vγ and 5 Jγ gene segments and 6 Vδ, 3 Dδ, and 4 Jδ gene segments (Figure 1).²⁻⁴

During T-cell differentiation in the thymus, immature T-cells form the V(D)J exons via rearrangement processes. For example, in the *TCRB* gene, Dβ to Jβ rearrangement generally occurs before Vβ to D-Jβ rearrangement, resulting in a specific V-D-Jβ exon that can be transcribed into *TCRB* mRNA and translated into TCRβ protein chains (Figure 2).⁵ During the rearrangement process, the DNA located between the rearranging gene segments is deleted from the genome and transformed into a so-called extrachromosomal (episomal) excision product (Figure 2), also called *"T-cell receptor excision circle"* (TREC).^{5,6} These episomal products cannot replicate in the cell and appear to be highly stable structures, which persist for a significant length of time.⁷ Comparable V-(D-)J rearrangement processes occur in the *TCRA, TCRG* and *TCRD* genes as well as in the Ig genes, which encode the Ig molecules of B-lymphocytes.²

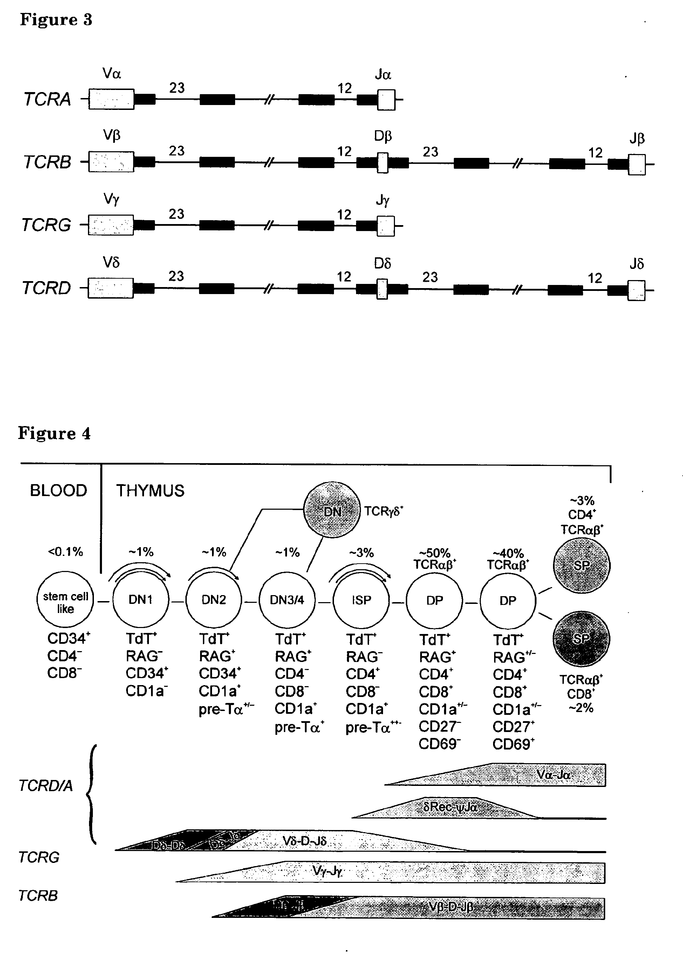

All V, D, and J gene segments are flanked by specific homologous recombination signal sequences (RSS). These RSS consist of a conserved palindromic heptamer sequence (CACAGTG) adjacent to the coding sequence and a conserved nonamer sequence (ACAAAAACC) that are separated by a less conserved spacer region of either 12 or 23 base pairs.^{6,8} In principle, only RSS of different spacer length join efficiently, known as the so-called 12/23 rule (Figure 3).⁸ RSS are recognized by recombination activation gene 1 and 2 proteins (RAG1 and RAG2), which are able to cleave the DNA between the RSS heptamer and the coding end of the involved gene segment.^{9,10} The DNA cleavage results in a hairpinned coding end and a blunt 5-phosphorylated signal end. A so-called coding joint is obtained after cleavage and ligation of the hairpinned coding ends. During this ligation process, further (junctional) diversity of the coding joints is obtained by deletion and insertion of nucleotides, resulting in a highly diverse junctional region. The signal ends are also ligated and thereby form the extrachromosomal circular excision product containing the two coupled RSS, which is referred to as the signal joint (Figure 2).

### TCR gene rearrangements during T-cell development

During T-cell differentiation in the thymus, the *TCRD* genes start to rearrange first: multistep rearrangement of Dδ to Dδ, DDδ to Jδ, and Vδ to DDJδ joinings. This is followed by the single step *TCRG* gene rearrangements with Vγ to Jγ joinings. If functionally rearranged *TCRD* and *TCRG* genes are obtained, TCRγδ protein molecules can be produced, allowing T-cells to further develop into the γδ T-cell lineage (Figure 4).^{11,12} T-cells with non-functional *TCRD*/*TCRG* gene rearrangements continue their TCR gene rearrangement process via the two step *TCRB* gene rearrangements (Dβ to Jβ followed by Vβ to DJβ joinings). This is followed by the complex rearrangement steps in the *TCRD*/*A* locus, i.e. the *TCRD* gene deletion (particularly by the δRec-ψJα rearrangement), and the subsequent single-step Vα-Jα rearrangement. If the *TCRB*/*TCRA* gene rearrangements are functional, TCRαβ protein molecules can be produced, allowing T-cells to further develop into αβ T-cell lineage (Figure 4).^{11,12}

### Peripheral T-cell expansion

Massive antigen-induced expansion of lymphocytes might theoretically become visible by the formation of a lymphocyte subpopulation with a highly-selected antigen-specific repertoire, but having a polyclonal origin. Such antigen-induced selection of lymphocytes is not (yet) detectable at the level of membrane-bound Ig molecules of B-lymphocytes, mainly because no suitable anti-Ig antibodies are available to reliably detect such antigen-selected subpopulations.

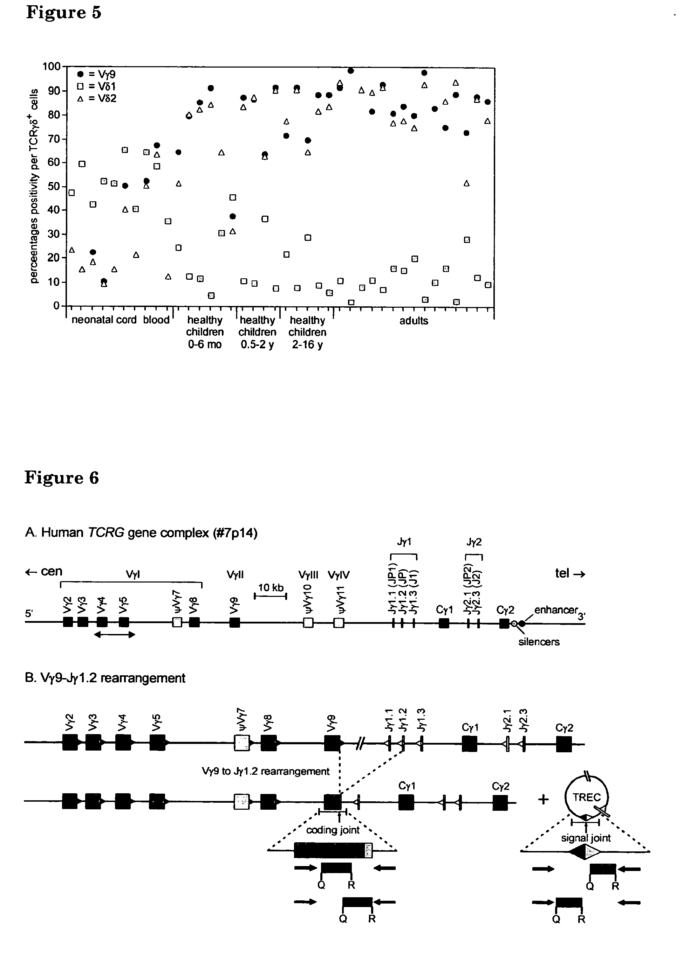

However, antigen-induced expansion and selection has been detected at the level of TCR molecules on T-lymphocytes by use of anti-Vα, anti-Vβ, anti-Vγ, and anti-Vδ antibodies. Two well-established examples have been described:
*1. Post-natal expansion of TCRγδ⁺ T-lymphocytes*
   The expansion of TCRγδ⁺ T-lymphocytes after birth mainly concerns T-cells with invariant expression of Vγ9-Jγ1.2 and Vδ2-Jδ1 receptors, which are apparently selected for their specificity for a superantigen.^{13,14} In neonatal cord blood and infancy, the Vγ9⁺/Vδ2⁺ T-lymphocytes represent only 5 to 15% of all TCRγδ⁺ T-lymphocytes. However in older children and adults 80 to 95% of TCRγδ⁺ T-lymphocytes in blood express the Vγ9/Vδ2 receptor (Figure 5).^{15,16} Remarkably, even the junctional region of the Vδ2-Jδ1 and Vγ9-Jγ1.2 rearrangements show a selection determinant.^{13,14} This implies that the selection and expansion of the Vγ9⁺/Vδ2⁺ T-cells is antigen-based. However, the extent of this expansion (the mean number of cell cycles reflecting the replicative history) is not known.
*2. Occurrence of specific natural killer cell-like T-lymphocytes (NKT-cells)*
   The vast majority of the so-called CD4-/CD8-/TCRαβ⁺ NKT-cells express an invariant TCR molecule, consisting of Vα18-Jα18 (previous codes: Vα24-JαQ), frequently found in combination with Vβ11.¹⁷⁻¹⁹ This invariant TCR molecule has a well-defined specificity for a-galactosylceramide (αGalCer).²⁰ The relative frequency of NKT-cells with Vα10-Jα18/Vβ11 expression is approximately 0.1 to 0.5% of T-lymphocytes.^{21,22} This might seem an extremely low frequency, but the random chance to produce T-lymphocytes with usage of the Vα10, Jα18, and Vβ11 gene segments is 1000-fold lower. This strongly suggests that the NKT-cells are not only antigen-selected, but probably have also expanded significantly. However, at present no information is available about *in vivo* NKT-cell proliferation.

The above two types of well-defined T-cell subsets can be detected and monitored by use of antibodies against their TCR chains: anti-Vγ9 and anti-Vδ2 for monitoring of the selected TCRγδ⁺ T-lymphocytes (see Figure 5)¹⁶; anti-Vα10 (Vα24) and anti-Vβ11 for detection and monitoring of the TCRαβ⁺ NKT-cells.^{22,23} However, precise measurement and evaluation of the Vγ9⁺/Vδ2⁺ expansions and the Vα10⁺/Vβ11⁺ NKT expansions needs molecular tools which can assess the mean number of cell cycles for each T-cell population.

Measuring the dilution of TREC's, such as the classically used δRec-ψJα signal joints, can show whether an αβ-lineage T-cell subset is expanded more than another αβ-lineage T-cell subset.^{7,24,25,26} However, the precise number of cell cycles can only be assessed if the number of episomal signal joints (no replication during cell cycle) can be compared with or normalized to the number of corresponding genomic coding joints (replication with each cell cycle). This approach is not possible for the δRec-ψJα signal joint, because the δRec-ψJα coding joint will be deleted upon a subsequent Vα-Jα rearrangement, thereby disqualifying the usage of δRec-ψJα coding joints as reliable controls for the accurate assessment of the mean number of cell cycles.^{7,25}

The limitations of the above methods known in the art prompted the present inventors to develop an improved episomal circular excision product - based detection method that allows for an accurate quantitation of the number of cell cycles in T- or B-cell subsets. The inventors now provide the insight that the targeted TCR or Ig gene rearrangement should be the last single step V to J (V-J) rearrangement of a TCR or Ig gene before obtaining the functional TCR or Ig molecule to ensure that no dilution of TREC's or BREC's will have taken place before completion of the total TCR or Ig gene rearrangement process. Furthermore, the so-called "end stage" TCR or Ig gene rearrangement ensures that the coding joint is retained in the genome of all cells of the target population and is not removed during a subsequent rearrangement (unlike e.g. the above mentioned rearrangement resulting in the δRec-ψJα coding joint and corresponding δRec-ψJα TREC).

Accordingly, the invention provides a method for determining the replicative history of a T-cell or a B-cell subset, comprising detecting a signal joint nucleotide sequence on a TREC (T-cell receptor excision circle) or a BREC (B-cell receptor excision circle) in at least one T-cell or one B-cell of said subset; and detecting a coding joint nucleotide sequence that is formed upon deletion of said TREC or BREC from a chromosome during a TCR or Ig gene rearrangement in said T-cell or B-cell, wherein said TCR or Ig gene rearrangement is the last single step V to J gene rearrangement before a functional TCR or Ig molecule is formed and wherein the chromosomal coding joint nucleotide sequence is stably retained in the chromosome.

The terms "replicative history" and "proliferative history" are used interchangeably. The replicative history of a T- or B-cell subset refers to the mean number of cell divisions which the cells within that subset have undergone since the production of a functional TCR or Ig molecule until the moment of analysis.

The detected chromosomal (genomic) coding joint sequence is replicated during cell division, whereas the episomal circular excision product (TREC or BREC) carrying the corresponding signal joint remains intact yet is not replicated. Consequently, with each round of replication of the cells present in the T- or B-cell subset of interest, the episomal products are diluted such that the coding/signal joint ratio increases. Therefore, in one embodiment of the invention, a method provided herein further comprising calculating the ratio between said chromosomal coding joint nucleotide sequence and said episomal (TREC or BREC) signal joint nucleotide sequence. The TCR gene rearrangement to be detected is preferably a *TCRG* (Vγ-Jγ) or a *TCRA* (Vα-Jα) rearrangement, as both the *TCRG* and the *TCRA* genes undergo functional rearrangement just prior to TCRγδ and TCRαβ expression on the cell surface, respectively (see also Figure 4). In another embodiment, the detected Ig gene rearrangement is an *IGK* (Vκ-Jκ) or an *IGL* (Vλ-Jλ) rearrangement. The targeted TCR or Ig gene rearrangement resulting from a single step V-J rearrangement allows easy design of primers and probes for real-time quantitative PCR (RQ-PCR) of both coding joints and the corresponding signal joints.^{27,28} Preferably, the targeted coding joint is of direct relevance for the Tor B-cell subset under study, meaning that the targeted TCR or Ig gene is coding for one of the expressed TCR / Ig protein chains.

The method of the invention and the disclosed oligonucleotide sequences (primers and probes) for use in a method of the invention provide unique possibilities to investigate the proliferative history and receptor diversity of lymphocyte subsets with a specific V-J rearrangement. By way of illustration, the invention is exemplified in relation to detecting the replicative history of two well known T-cell subsets: Vγ9-Jγ1.2⁺ T-lymphocytes and Vα10-Jα18⁺ NKT-cells. However, the skilled person will recognize that the teaching of the present invention is applicable for any specific V-J rearrangement expressed in T-lymphocytes (Vα-Jα or Vγ-Jγ) and B-lymphocytes (Vκ-Jκ or Vλ-Jλ), as long as single step rearrangements are used as PCR targets with application of gene-segment-specific primers and probes, which do not cross-anneal to other gene segments.

The Vγ9-Jγ1.2 rearrangements in the antigen-selected TCRγδ⁺ T-lymphocyte and the Vα10-Jα18 (Vα24-JαQ) rearrangements in NKT-cells fulfil all above requirements and can therefore be regarded as excellent RQ-PCR targets for assessment of the replicative history of Vγ9-Jγ1.2 expressing T-lymphocytes and Vα10-Jα18 expressing NKT-cells. Vγ9-Jγ1.2 rearrangements are extremely rare in non-TCRγδ T-lymphocytes and are virtually exclusively found in Vγ9⁺/Vδ2⁺ T-lymphocytes.^{29,30} Also Vα10-Jα18 rearrangements are rare (if present at all) in non-NKT-cells.^{17,21,22}

### Replicative history of Vγ9⁺/Vδ2⁺ T-lymphocytes

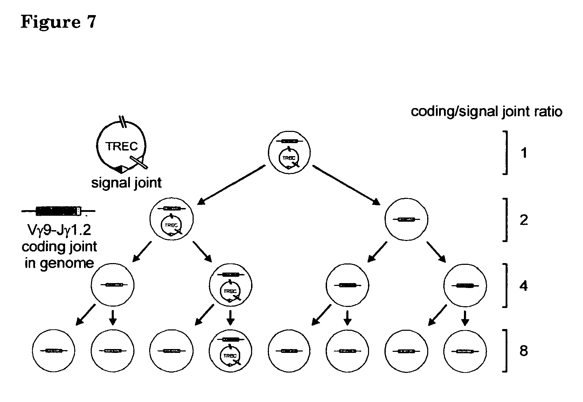

Since Vγ9-Jγ1.2 rearrangements are virtually exclusively found in the antigen-selected Vγ9⁺/Vδ2⁺ blood T-lymphocytes, it is possible to use RQ-PCR analysis of the Vγ9-Jγ1.2 coding joint and the corresponding signal joint to study the replicative history of these T-cells (Figure 6).^{27,28,31} The coding joint/signal joint ratio corresponds to the mean offspring per Vγ9-Jγ1.2 expressing target cell and is related to the mean number of cell cycles (Figure 7).

The person skilled in the art will recognize that several primer/probe combinations can be designed for RQ-PCR detection and quantitation of the Vγ9-Jγ1.2 coding joints and signal joints (Table 1).^{27,28,31} It is also possible to design a probe or a primer at the Vγ9-Jγ1.2 junctional region (Figure 6), because many Vγ9-Jγ1.2 expressing T-lymphocytes have an invariant canonical Vγ9-Jγ1.2 junctional region, caused by a biased recombination process via homologous sequences (GCA) at the Vγ9 and Jγ1.2 coding ends.^{13,14}

As Vγ9-Jγ1.2 rearrangements are extremely rare in non-Vγ9⁺/Vδ2⁺ T-cells, it is generally not necessary to purify the Vγ9⁺/Vδ2⁺ T-cells for DNA extraction to evaluate their replicative history. In fact, the total mononuclear cell (MNC) population or the total blood T-cell population might be used for DNA extraction.
Accordingly, the invention provides as a specific embodiment a method for determining the replicative history of Vγ9-Jγ1.2⁺ T lymphocytes, comprising 1) detecting a signal joint nucleotide sequence on a TREC (T-cell receptor excision circle) in at least one T-cell of said subset; 2) detecting a coding joint nucleotide sequence that is formed upon deletion of said TREC from a chromosome during a TCR rearrangement in said T-cell, wherein said TCR rearrangement is the Vγ9-Jγ1.2 rearrangement.

### Replicative history of Vα10⁺/Vβ11⁺ NKT-cells

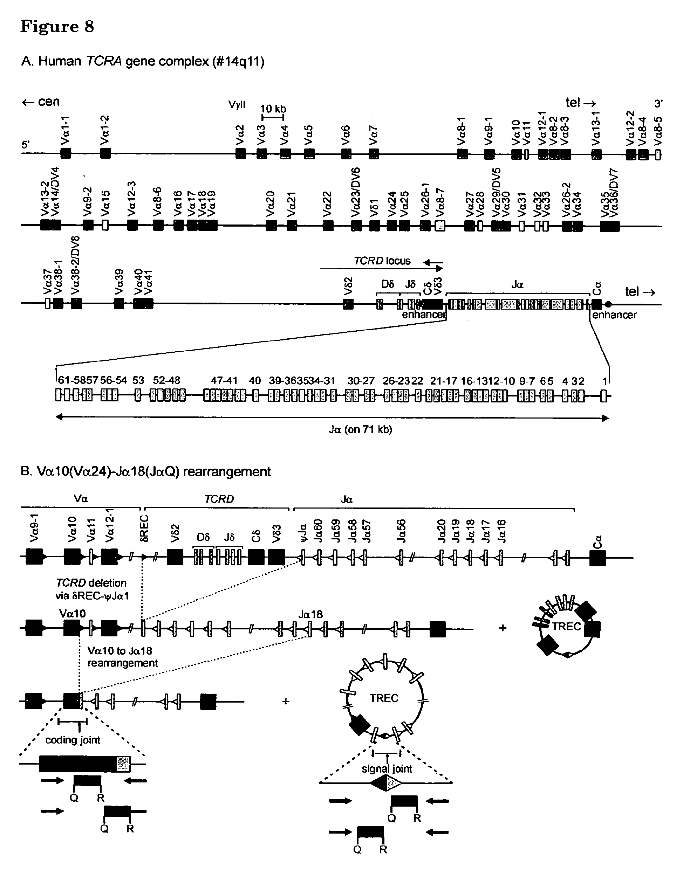

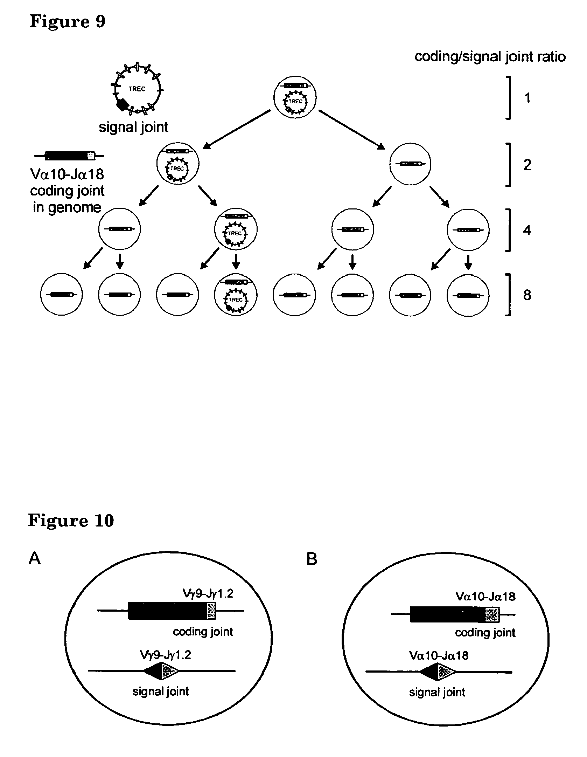

The Vα10-Jα18 rearrangement is virtually exclusively found in the Vα10⁺/Vβ11⁺ NKT-cells. Therefore RQ-PCR analysis of the Vα10-Jα18 coding joints and signal joints can be used to assess the replicative history of the NKT-cells (Figure 8).^{27,28} The coding joint/signal joint ratio corresponds to the mean offspring per Vα10-Jα18 expressing NKT-cell and is related to the mean number of cell cycles (Figure 9).

Several primer/probe combinations can be designed for RQ-PCR detection and quantitation of the Vα10-Jα18 coding joints and signal joints (Table 2). It might even be possible to design a probe or primer at the Vα10-Jα18 junctional region (Figure 8), because a part of the Vα10-Jα18 rearrangements have junctional regions without N region insertion.^{17,18} Since Vα10-Jα18 rearrangements are extremely rare in non-NKT-cells, it is probably not necessary to purify the NKT-cells for studying their replicative history. Even though the frequency of NKT cells generally is <1%, RQ-PCR can evaluate the replicative history of NKT-cells in total leukocyte DNA.

Thus, in another specific embodiment the invention provides a method for determining the replicative history of NKT-cells, comprising 1) detecting a signal joint nucleotide sequence on a TREC (T-cell receptor excision circle) in at least one T-cell of said subset; 2) detecting a coding joint nucleotide sequence that is formed upon deletion of said TREC from a chromosome during a TCR rearrangement in said T-cell, wherein said TCR rearrangement is the Vα10-Jα18 rearrangement.

Detection of a coding joint nucleotide sequence and a signal joint nucleotide sequence in a method of the invention can be performed using conventional molecular biological procedures. Preferably, it involves PCR analysis, more preferably RQ-PCR analysis. RQ-PCR permits accurate quantitation of PCR products during the exponential phase of the PCR amplification process, which is in full contrast to the classical PCR end point quantitation. Owing to the real-time detection of fluorescent signals during and/or after each subsequent PCR cycle, quantitative PCR data can be obtained in a short period of time and no post-PCR processing is needed, thereby drastically reducing the risk of PCR product contamination. RQ-PCR technology for example can use an ABI Prism 7700 instrument (TaqMan®) to detect accumulation of PCR products continuously during the PCR process thus allowing easy and accurate quantitation in the early exponential phase of PCR. The ABI Prism 7700 uses fiber optic systems, which connect to each well in a 96-well PCR tray format. A laser light source excites each well and a CCD camera measures the fluorescence spectrum and intensity from each well to generate real-time data during the PCR amplification process. The ABI 7700 Prism software examines the fluorescence intensity and calculates the increase in intensity over the course of the amplification. The results are then plotted versus cycle number, to produce a continuous measure of PCR amplification. To provide precise quantification of the initial target in each PCR, the amplification plot is examined at a point during the early log phase of product accumulation. This is accomplished by assigning a fluorescence threshold above background and determining the PCR cycle at which each sample's amplification plot reaches the threshold (defined as the threshold cycle number or CT). PCR products can of course also be analyzed by standard gel detection methods, for example using heteroduplex analysis with ethidium staining. However, for quantification of the amplified coding joint/signal joint products and the calculation of their ratio, the use of quantitative methods such as RQ-PCR are preferred.

At present three main types of RQ-PCR techniques are available. In one embodiment of the invention, detection is based on detection of PCR products by the intercalating dye SYBR Green I. This dye can bind to the minor groove of double-stranded DNA, which greatly enhances its fluorescence. During the consecutive PCR cycles, the amount of double stranded PCR product will exponentially increase, and therefore more SYBR Green I dye can bind and emit its fluorescence (at 520 nm). It should be noted that SYBR Green I-based detection of PCR products is not sequence specific and that consequently also non-specifically amplified PCR products and primer dimers will be detected. In addition to SYBR-Green I, also other dyes can be used in non-specific detection systems such as Amplifluor.

In a preferred embodiment, a method of the invention comprises detection and quantitation of a chromosomal coding joint or signal joint nucleotide sequence using RQ-PCR with hydrolysis probes. This type of RQ-PCR exploits the 5' → 3' exonuclease activity of the *Thermus aquaticus (Taq)* polymerase to detect and quantify specific PCR products as the reaction proceeds. The hydrolysis probe, also referred to as TaqMan probe or double-dye oligonucleotide probe, is conjugated with a reporter (R) fluorochrome (e.g. FAM, VIC or JOE) as well as a quencher (Q) fluorochrome (e.g. TAMRA) and should be positioned within the target sequence. The quencher fluorochrome absorbs the fluorescence of the reporter fluorochrome as long as the probe is intact. However, upon amplification of the target sequence, i.e. the coding joint or the signal joint sequence, the hydrolysis probe is displaced and hydrolysed by the *Taq* polymerase. This results in the separation of the reporter and quencher fluorochrome and consequently the fluorescence of the reporter fluorochrome becomes detectable. During each consecutive PCR cycle this fluorescence will further increase because of the progressive and exponential accumulation of free reporter fluorochromes.

In yet a further embodiment, RQ-PCR using hybridisation probes is used for the detection and quantitation of a coding joint sequence and a signal joint sequence of interest in a T-cell, such that the replicative history of the T-cell subset can be determined. RQ-PCR analysis with hybridisation probes uses two juxtaposed sequence-specific probes, one labelled with a donor fluorochrome (e.g. FAM) at the 3' end and the other labelled with an acceptor fluorochrome (e.g. LC Red640, LC Red705) at its 5' end. Both probes should hybridise to closely juxtaposed target sequences on the amplified DNA fragment, thereby bringing the two fluorochromes into close proximity (preferably within 1-5 nucleotides) such that the emitted light of the donor fluorochrome will excite the acceptor fluorochrome. This results in the emission of fluorescence, which can be detected during the annealing phase and the first part of the extension phase of the PCR reaction. After each subsequent PCR cycle, more hybridisation probes can anneal, resulting in higher fluorescence signals.

In addition to the three main RQ-PCR approaches described above, other types of probes may also be used in a method provided herein, including molecular beacons, Scorpions, minor groove-binding (MGB) probes, ResonSense, Hy-Beacon, and Light-up probes.

In another aspect, the invention provides a set of at least two pairs of nucleic acid amplification primers comprising at least a first pair of primers for detecting a signal joint nucleotide sequence on a TREC (in case of T-cells) or a BREC (in case of B-cells) and a second pair of primers for detecting a chromosomal coding joint nucleotide sequence in a T- or B-cell subset, wherein said TREC or BREC and said coding joint nucleotide sequence are formed during the last single step V-J TCR or Ig gene rearrangement before a functional TCR or Ig molecule is formed and wherein the chromosomal coding joint nucleotide sequence is stably retained in the chromosome, preferably because the coding joint is included in the expressed TCR or Ig gene.

In one embodiment, such a set comprises a pair of primers for detecting a signal joint nucleotide sequence on a TREC that is deleted from a chromosome during a *TCRG* or a *TCRA* rearrangement, as well as a pair of primers to detect the corresponding chromosomal coding joint sequence. In another embodiment, the set comprises a pair of primers for detecting a signal joint nucleotide sequence on a BREC that is deleted from a chromosome during a *IGK* or an *IGL* rearrangement, as well as a pair of primers to detect the corresponding chromosomal coding joint sequence. If the TCR repertoire of a T-cell subset of interest, or the Ig repertoire of a B-cell subset of interest, is known, the primers can be designed on the basis of the gene segments of the rearranged locus. The primers are designed to detect the coding and signal joint sequences that have resulted from a single step V to J rearrangement, for example a Vα-Jα or Jγ-Jγ rearrangement (T-cells) or a Vκ-Jκ or Vλ-Jλ rearrangement (B-cells). Once the T-cell subset of interest is defined and once the involved Vα and Jα gene segments or the involved Vγ and Jγ gene segments of the target population have been identified, the skilled person will be able to design the suitable sets of primers e.g. using publicly available TCR gene sequences and primer design software. Similarly, specific primers for detecting Vκ-Jκ en Vλ-Jλ rearrangements in B cells can be readily designed. In specific embodiments, the primer set is designed to detect the coding and signal joint sequences that are the result of the Vγ9-Jγ1.2 rearrangement or the Vα10-Jα18 rearrangement. The invention provides a primer having a nucleotide sequence selected from the group of oligonucleotide primers presented in Tables 1 and 2, or a variant thereof.

The term "variant" refers to a primer which differs in 1 to 5 nucleotides, preferably 1 to 3 nucleotides, from the size and/or position of a primer sequence shown in Table 1 or Table 2, provided that the nucleotide sequence of said variant primer contains at most 2 mismatches, preferably at most 1 mismatch, most preferably no mismatches with the target sequence and that the variant primer hybridises with the target nucleotide sequence. In addition, a variant primer comprises a (differentially) labeled primer, i.e. a primer having a label that can be identified or distinguished from other labels by any means, including the use of an analytical instrument. Examples of differentially labeled primers are primers provided with a fluorescent label such as a 6-FAM, HEX, TET or NED dye. In a specific embodiment, a set of primers of the invention comprises at least one of the primers selected from those set out in Tables 1 and 2, or a variant thereof.

In yet another aspect of the invention, a nucleic acid amplification assay, preferably a PCR assay, more preferably a RQ-PCR assay is provided using a set of primers as provided herein. For example, a two tube PCR assay is provided wherein one tube comprises a set of a forward and a reverse primer for amplification of a coding joint sequence of an end-stage TCR or Ig gene rearrangement in a T-cell or a B-cell and wherein another tube comprises a set of a forward and a reverse primer for amplification of the corresponding TREC or BREC. Preferably, the amplification assay comprises amplification of the signal joint and the coding joint sequences that are the result of a single step V-J rearrangement, like a Vα-Jα or a Vγ-Jγ rearrangement in case of a T-cell or a Vκ-Jκ or Vλ-Jλ rearrangement in case of a B-cell. In one embodiment, the invention provides a nucleic acid amplification assay using a set of primers designed for amplification of the Vα10-Jα18 coding joint and signal joint nucleotide sequences, for example using primers as presented in Table 2. This assay is advantageously used to assess the replicative history of NKT-cells. In another embodiment, the invention provides a nucleic acid amplification assay using a set of primers designed for amplification of the Vγ9-Jγ1.2 coding joint and signal joint nucleotide sequences, for example using primers as presented in Table 1.

**TABLE 1. Primers and probes for RQ-PCR detection of coding and signal joints of Vγ9-Jγ1.2 rearrrangements**

| **Oligonucleotides for RQ-PCR** | | | |
|---|---|---|---|
| **Type** | **Code** | **5' sequence 3'** | **5' Position from RSS** |
| **Vγ9-Jγ1.2 coding joint** | | | |
| Primer | Vγ9 RQ-F-1 (set 1) | CATTCACAATGTAGAGAAACAGGACA | -57 |
| Primer | Jγ1.2 RQ-R-1 (set 1) | AAGAAAACTTACCTGTAATGATAAGC | +72 |
| Probe | Tr Vγ9-Jγ1.2 pan-1 (set 1) | TTGTTCCGGGACCAAATACCTTGATT | +45 |
| Primer | Vγ9 RQ-F-2 (set 2) | GGTGGATAGGATACCTGAAACGTC | -96 |
| Primer | Jγ1.2 RQ-R-2 (set 2) | CAGCCTCCCATCCCTTCTTTAC | +106 |
| Probe | T Vγ9-Jγ1.2 pan-2 (set 2) | TGGTCCCGGAACAAAGCTTATCATTACAGG | +59 |
| Probe | Tr Vγ9-Jγ1.2 can (set 1 and 2) | TGCCCAACTCTTGCACCTCCCAC | At canonical junction |

| **Vγ9-Jγ1.2 signal joint** | | | |
|---|---|---|---|
| Primer | Vγ9 TREC-F-1 (set 1) | CTGTGGCTTCCCAATTCGTAA | +84 |
| Primer | Jγ1.2 TREC-R-1 (set 1) | CGTCAGCAGGGCCAATTTT | -107 |
| Probe | T Vγ9-Jγ1.2 TREC-1 (set 1) | TTGGCACAATTTTAAGACAAACACAAAGGGAG | -60 |
| Primer | Vγ9 TREC-F-2 (set 2) | TGAATGGGATGGCTCAAACTG | +33 |
| Primer | Jγ1.2 TREC-R-2 (set 2) | AGGGCCAATTTTTATGTATTTATGTAA | -101 |
| Probe | Tr Vγ9-Jγ1.2 TREC-2 (set 2) | AGAAGCCTTTATAAGAATCTCCCTTTGTGTTTGTCT | -46 |

**TA8LE 2. Primers and probes for RQ-PCR detection of coding and signal joints of Vα10-Jα18 rearrrangements**

| **Oligonucleotides for RQ-PCR** | | | |
|---|---|---|---|
| **Type** | **Code** | **5' sequence 3'** | **5^{,} Position from RSS** |
| **Vα10-Jα18 coding joint** | | | |
| Primer | Vα10 RQ-F-1 (set 1) | ATCACAGCCTCCCAGCTCAG | -51 |
| Primer | Jα18 RQ-R-1 (set 1) | ATTTTCCTGGAATAGAAAGCGACTC | +95 |
| Probe | T Vα10-Jα18 pan-1 (set 1) | AGGAACTCAGTTGACTGTCTGGCCTGGTG | +67 |
| Primer | Vα10 RQ-F-2 (set 2) | CGGAAGATATACAGCAACTCTGGAT | -103 |
| Primer | Jα18 RQ-R-2 (set 2) | TGGAATAGAAAGCGACTCACTCAC | +88 |
| Probe | Tr Vα10-Ja18 pan-2 (set 2) | GCCAGACAGTCAACTGAGTTCCTCTTCCAAAGTA | +61 |
| Probe | Tr Vα10-Jα18 inv (set 1 and 2) | AGCCTCTGTCGCTCACCACACAGATG | At blunt (invariant) junction |

| **Vα10-Jα18 signal joint** | | | |
|---|---|---|---|
| Primer | Vα10 TREC-R-1 (set 1) | CCTTGATAACCTCCAGGTGCAG | +60 |
| Primer | Jα18 TREC-F-1 (set 1) | GCTTAAGGAGGATGGAGGCAA | -106 |
| Probe | T Vα10-Jα18 TREC-1 (set 1) | ATGGGAAACATTAGGGCTGGGTTCATGT | -47 |
| Primer | Vα10 TREC-R-2 (set 2) | CTGCATAAAAAATAACGGTCTACTCTAACT | + 99 |
| Primer | Jα18 TREC-F-2 (set 2) | AGGATGGAGGCAAATTTTCAAC | -106 |
| Probe | Tr Vα10-Jα18 TREC-2 (set 2) | GTTTCCCATCCCCTCTCCCCTCAGC | -63 |

Also provided herein is an oligonucleotide probe that is homologous to an internal sequence of an amplified nucleic acid sequence (amplicon) produced in a nucleic acid amplification assay according to the invention to detect and quantify such an amplicon. Such a probe may be non-specific or sequence-specific, and they may be provided with at least one, preferably two fluorochromes, such as hydrolysis probes or hybridisation probes. Examples of useful sequence-specific probes according to the invention are depicted in Tables 1 and 2. These probes, or variants thereof (for definition of the term 'variant' see above) are advantageously used to detect and quantify the coding joint nucleotide sequence and the signal joint nucleotide sequence in a T-cell or B-cell, such that the ratio can be calculated between the chromosomal (replicating) coding joint and the TREC or BREC (non-replicating) signal joint sequence.

Importantly, the invention also provides a T-cell or B-cell (or a culture or stock T-cell line or B-cell line thereof), which can be used as a positive control cell in a method of the invention. This cell is a T-cell or B-cell comprising a coding joint nucleotide sequence that is stably retained in the chromosome and that is the result of the last single step TCR or Ig gene rearrangement before a functional TCR or Ig molecule is formed. Importantly, the T-cell or B-cell is stably transformed (e.g. using retroviral gene transduction) with one copy of a recombinant nucleic acid sequence that is homologous to the corresponding signal joint nucleotide sequence that is formed during the TCR or Ig gene rearrangement.

In contrast to the T-cells or B-cells to be tested, the coding joint and the signal joint sequences that are present in a control cell line according to the invention are both replicated during cell division. Consequently, the ratio between the two can be set at 1.0. A control cell as provided herein not only serves as an internal control for the ratio between the coding joint and signal joint sequence, but also as 100% setting for the (RQ)-PCR reactions that are performed to detect and quantify the presence of the specific coding joint and signal joint sequences in a T-cell or B-cell population.

In one aspect the control T-cell comprises in its genome the coding joint nucleic acid sequence and the corresponding signal joint nucleotide sequence formed during the Vγ9-Jγ1.2 or the Vα10-Jα18 rearrangement. These cells are very convenient as positive controls in reliable RQ-PCR studies on Vγ9-Jγ1.2 and Vα10-Jα18 coding and signal joints, respectively. Optimal positive controls can be obtained by selection of a Vγ9-Jγ1.2 containing T-cell line or a Vα10-Jα18 containing T-cell line and by transduction of these cell lines with retroviral constructs that contain the respective corresponding signal joint (see Figure 10). Of course, control B-cells or B-cell lines can be generated according to the same principles.

A further aspect of the invention relates to diagnostic kits comprising means for carrying out a method according to the invention. Such a kit comprises for example a primer or probe of the invention (see Tables 1 and 2), and/or a set of at least two pairs of nucleic acid amplification primers comprising at least a first pair of primers for detecting a signal joint nucleotide sequence on a TREC (T-cell receptor excision circle) or on a BREC (B-cell receptor excision circle) and a second pair of primers for detecting the corresponding chromosomal coding joint nucleotide sequence in a T-cell subset or B-cell subset, wherein said TREC or BREC and said corresponding coding joint nucleotide sequence are formed during the last single step V to J gene rearrangement before a functional TCR or Ig molecule is formed and wherein the chromosomal coding joint nucleotide sequence is stably retained in the chromosome. Examplary primers to be part of a set of primers are those set out in Tables 1 and 2. However, it will be understood that for the detection of any specific T- or B-cell subset of interest the oligonucleotides in the kit will be adjusted to the specific target sequences to be detected. The skilled person will be able to select the TCR and Ig gene target sequences and design suitable nucleic acid amplification primers.

Preferably, the kit further contains one or more detection probes for detecting the amplified sequence, for example a TaqMan probe, which allows detection and quantitation of an end-stage coding joint sequence and/or a probe for detection and quantitation of TRECs or BRECs containing the corresponding signal joint sequence. Furthermore, a kit of the invention may comprise a control cell according to the invention and/or instructions for use of the kit components in a nucleic acid amplification assay (see refs. 27, 28, and 32 for details). A kit can of course also comprise buffers, such as a buffered magnesium solution with deoxynucleotides, diluents and/or an enzyme, for example *Taq* polymerase.

A method of the invention to determine the replicative history of a T-cell or B-cell subset of interest has many important clinical and scientific applications. It is for example useful for investigating the replicative history of T-cell and B-cell expansions in immunological and hematological diseases.

In a specific aspect, a method of the invention is used in relation to junctional diversity, which is the variability in immunoglobulins caused by differences in the exact crossover point during V-J, V-D, and D-J joinings. Massive antigen-induced proliferation of a specific T-cell subset theoretically can result in a reduction of the antigen receptor repertoire. However, the expanded Vγ9-Jγ1.2⁺ T-lymphocytes and the expanded Vα10-Jα18⁺ NKT-cells can still have diverse junctional regions, despite the fact that a part of the Vγ9-Jγl.2 rearrangements have canonical junctions and that a part of the Vα10-Jα18⁺ NKT-cells have junctional regions without N region insertion. 13,14,17,18 The junctional region diversity can easily be checked by GeneScan analysis of PCR products of the Vγ9-Jγ1.2 and Vα10-Jα18 rearrangements.⁵ Thus, analysis of V-J coding joints/signal joints as disclosed herein is advantageously combined with GeneScanning of the same V-J-specific rearrangements to determine the effect of antigen-induced proliferation on junctional diversity.

### LEGENDS TO THE FIGURES

**Figure 1: Schematic diagram of human T-cell receptor gene complexes.**
   The *TCRA* gene complex consists of approximately 46 functional Vα gene segments, a stretch of 53 functional Jα gene segments, and one C gene segment. The *TCRB* gene complex contains approximately 47 functional Vβ gene segments and two C gene segments, both of which are preceded by a Dβ gene segment and six or seven Jβ gene segments. The *TCRG* gene complex consists of a restricted number of Vγ gene segments (six functional segments and three rearrangable pseudo-segments) and two C gene segments, each preceded by two to three Jγ gene segments. The *TCRD* gene complex comprises several V (three true Vδ segments and several Vα/Vδ segments), three D, four J, and one C gene segment. The major part of the *TCRD* gene complex is located between the Vα and Jα gene segments and is flanked by the δRec and ψJa gene segments, which are involved in deletional *TCRD* gene rearrangements that occur before *TCRA* gene rearrangements. Pseudogenes (ψ) are indicated as open symbols.
**Figure 2: Schematic diagram of sequential rearrangement steps, transcription, and translation of the *TCRB* gene.**
   In this example, first a Dβ to Jβ rearrangement occurs, followed by Vβ to Dβ-Jβ rearrangement, resulting in the formation of a Vβ-Dβ-Jβ exon with a junctional region, that contains two coding joints.⁵ The rearranged *TCRB* gene is transcribed into precursor mRNA, spliced into mature mRNA, and finally translated into TCRβ protein. The two extrachromosomal T-cell receptor excision circles (TRECs) that are formed during this recombination process are depicted as well; they contain the D-J signal joint and V-D signal joint, respectively.
**Figure 3: Recombination signal sequences of human TCR genes.**
   The vast majority of functional RSS consist of a conserved palindromic heptamer sequence adjacent to the coding sequence and a conserved nonamer sequence that are separated by a less conserved spacer region of either 12 or 23 base pairs. In principle, only RSS of different spacer length join efficiently, known as the so-called 12/23 rule.⁸
**Figure 4: Hierarchical order of TCR gene rearrangement processes during human T-cell development.^{2,11,12}**
   The *TCRD* genes start to rearrange first: Dδ to Dδ, Dδ-Dδ to Jδ, and Vδ to Dδ-Dδ-Jδ. This is followed by *TCRG* gene rearrangements (Vγ-Jγ joinings). Functional *TCRG* and *TCRD* gene rearrangements result in TCRγδ⁺ T-cells. T-cells of the αβ lineage proceed with *TCRB* gene rearrangements (Dβ to Jβ, followed by Vβ to Dβ-Jβ joinings). Subsequent rearrangements in the *TCRA*/*D* locus concern the *TCRD* gene deletion (particularly via δRec-ψJα rearrangement) and the Vα-Jα rearrangement. Functional *TCRA* and *TCRB* rearrangements result in TCRαβ⁺ T-cells.
**Figure 5 : V gene segment usage by TCRγδ⁺ T-lymphocytes in blood from neonates, children, and adults.**
   Vγ9, Vδ1, and Vδ2 protein expression was determined by immunofluorescence stainings (Ti-γA, δTCS1, and BB3 antibodies, respectively). A major shift in V gene segment usage was observed from predominant Vδ1 expression (~60%) and limited Vγ9 and Vδ2 expression in neonates and young children to predominant Vγ9 and Vδ2 expression (80 to 95%) in older children and adults. This shift to Vγ9⁺/Vδ2⁺ T-lymphocytes was proven to be caused by an antigen-induced selection and expansion of TCRγδ⁺ T-lymphocytes.13,16
**Figure 6 : Human *TCRG* gene complex and Vγ9-Jγ1.2 rearrangement with RQ-PCR detection of coding and signal joints.**
   *Panel A.* The human *TCRG* locus consists of nine Vγ gene segments and two Cγ gene segments, each preceded by two or three Jγ gene segments.
   *Panel B.* The Vγ9-Jγ1.2 rearrangement occurs at high frequency in blood TCRγδ⁺ T-lymphocytes. The genomic Vγ9-Jγ1.2 coding joint and the episomal Vγ9-Jγ1.2 signal joint can de detected and quantified by use of RQ-PCR, for example using the TaqMan technology. The position of the primers and TaqMan probes is indicated. Sequences of the primers and probes are provided in Table 1.
**Figure 7 : Coding/signal joint ratios during consecutive divisions of Vγ9-Jγ1.2⁺ T-cells.**
   With each round of replication, the chromosomal Vγ9-Jγ1.2 coding joint in a Vγ9-Jγ1.2⁺ T-cell is replicated whereas the episomal circular excision product carrying the Vγ9-Jγ1.2 signal joint remains intact yet is not replicated. Consequently, with each round of replication of Vγ9-Jγ1.2⁺ T-cells, the episomal products are diluted during division such that the coding/signal joint ratio progressively increases.
**Figure 8 : Human *TCRA* gene complex and Vα10-Jα18 rearrangement with RQ-PCR detection of coding and signal joints.**
   *Panel A.* The *TCRA* locus contains approximately 46 functional Vα gene segments and 53 functional Jα segments. The major part of the *TCRD* locus is located within the *TCRA* locus between the Vα and Jα gene segments and flanked by the *TCRD* deleting elements δRec and ψJα. *Panel B.* Vα-Jα rearrangements are preceded by deletion of the *TCRD* locus, generally via δRec-ψJα rearrangement. The depicted Vα10-Jα18 rearrangement is accompanied by the formation of a TREC with the corresponding signal joint. The genomic Vα10-Jα18 coding joint and the episomal Vα10-Jα18 signal joint can be detected and quantified by use of RQ-PCR, for example using the TaqMan technology. The position of the primers and TaqMan probes is indicated. Sequences of the primers and probes are provided in Table 2.
**Figure 9: Coding/signal joint ratios during consecutive divisions of Vα10-Jα18⁺ NKT-cells.**
   With each round of replication, the chromosomal Vα10-Jα18 coding joint in an NKT-cell is replicated whereas the episomal circular excision product carrying the Vα10-Jα18 signal joint remains intact yet is not replicated. Consequently, with each round of replication of an NKT-cell, the episomal products are diluted during division such that the coding/signal joint ratio progressively increases.
**Figure 10 :**
   Schematic representation of the control cell lines for RQ-PCR analysis of Vγ9-Jγ1.2 rearrangements (Panel A) and Vα10-Jα18 rearrangements (Panel B). The control cell lines contain the Vγ9-Jγ1.2 or Vα10-Jα18 coding joint, respectively, and are stably transformed with the sequence of the corresponding signal joint. In contrast to T-lymphocytes, the coding and signal joints in the two control cell lines are both replicated during cell division, thereby guaranteeing that the coding joint/signal joint ratio of the cell lines can be set at 1.0.

### REFERENCES

1. Comans-Bitter WM, De Groot R, Van den Beemd MWM, Neijens HJ, Hop WCJ, Groeneveld K, Hooijkaas H, Van Dongen JJM. Immunophenotyping of blood lymphocytes in childhood: Reference values for lymphocyte subpopulations. J Pediatr 1997;130:388-393.
2. Van Dongen JJM, Wolvers-Tettero ILM. Analysis of immunoglobulin and T cell receptor genes. Part I: Basic and technical aspects. Clin Chim Acta 1991;198:1-91.
3. Davis MM, Björkman PJ. T-cell antigen receptor genes and T-cell recognition. Nature 1988;334:395-402.
4. Lefranc MP. IMGT databases, web resources and tools for immunoglobulin and T cell receptor sequence analysis, http://imgt.cines.fr. Leukemia 2003;17:260-266.
5. Van Dongen JJM, Langerak AW, Brüggemann M, Evans PA, Hummel M, Lavender L, Delabesse E, Davi F, Schuuring E, Garcia Sanz R, Van Krieken JHJM, Droese J, Gonzalez Diaz D, Bastard D, White H, Spaargaren M, Gonzáles M, Parreira A, Smith J, Morgan G, Kneba M, Macintyre EA. Design and standardization of PCR primers and protocols for detection of clonal immunoglobulin and T-cell receptor gene recombinations in suspect lymphoproliferations. Report of the BIOMED-2 Concerted Action BMH4-CT98-3936. Leukemia 2003;17:2257-2317.
6. Lieber MR. The mechanism of V(D)J recombination: a balance of diversity, specificity, and stability. Cell 1992;70:873-876.
7. Hazenberg MD, Verschuren MCM, Hamann D, Miedema F, Van Dongen JJM. T cell receptor excision circles (TRECS) as markers for recent thymic emigrants. Basic aspects, technical approach, and guidelines for interpretation. J Mol Med 2001;79:631-640.
8. Van Gent DC, Ramsden DA, Gellert M. The RAG1 and RAG2 proteins establish the 12/23 rule in V(D)J recombination. Cell 1996;85:107-113.
9. Schatz DG, Oettinger MA, Baltimore D. The V(D)J recombination activating gene, RAG-1. Cell 1989;59:1035-1048.
10. Oettinger MA, Schatz DG, Gorka C, Baltimore D. RAG-1 and RAG-2, adjacent genes that synergistically activate V(D)J recombination. Science 1990;248:1517-1523.
11. Van Dongen JJM, Comans-Bitter WM, Wolvers-Tettero ILM, Borst J. Development of human T lymphocytes and their thymus-dependency. Thymus 1990;16:207-234.
12. Dik WA, Pike-Overzet K, Weerkamp F, de Ridder D, de Haas EF, Baert MR, van der Spek P, Koster EE, Reinders MJ, van Dongen JJ, Langerak AW, Staal FJ. New insights on human T cell development by quantitative T cell receptor gene rearrangement studies and gene expression profiling. J Exp Med 2005;201:1715-1723.
13. Breit TM, Wolvers-Tettero ILM, Van Dongen JJM. Unique selection determinant in polyclonal Vδ2-Jδ1 junctional regions of human peripheral γδ T-lymphocytes. J Immunol 1994;152:2860-2864.
14. Davodeau F, Peyrat MA, Hallet MM, Houde I, Vie H, Bonneville M. Peripheral selection of antigen receptor junctional features in a major human gamma delta subset. Eur J Immunol 1993;23:804-808.
15. Parker CM, Groh V, Band H, Porcelli SA, Morita C, Fabbi M, Glass D, Strominger JL, Brenner MB. Evidence for extrathymic changes in the T cell receptor gamma/delta repertoire. J Exp Med 1990;171:1597-1612.
16. Sandberg Y, Almeida J, Gonzalez M, Lima M, Barcena P, Szczepanski T, Van Gastel-Mol EJ, Wind H, Balanzategui A, Van Dongen JJM, Miguel JF, Orfao A, Langerak AW. TCRγδ+ large granular lymphocyte leukemias reflect the spectrum of normal antigen-selected TCRγδ+ T-cells. Leukemia 2006;20:505-513.
17. Dellabona P, Padovan E, Casorati G, Brockhaus M, Lanzavecchia A. An invariant Vα24-JαQ/Vβ11 T cell receptor is expressed in all individuals by clonally expanded CD4-8- T cells. J Exp Med 1994;180:1171-1176.
18. Han M, Harrison L, Kehn P, Stevenson K, Currier J, Robinson MA. Invariant or highly conserved TCRαare expressed on double-negative (CD3+CD4-CD8-) and CD8+ T cells. J Immunol 1999;163:301-311.
19. Kronenberg M. Toward an understanding of NKT cell biology: progress and paradoxes. Annu Rev Immunol 2005;23:877-900.
20. Sidobre S, Hammond KJ, Benazet-Sidobre L, Maltsev SD, Richardson SK, Ndonye RM, Howell AR, Sakai T, Besra GS, Porcelli SA, Kronenberg M. The T cell antigen receptor expressed by Vα14i NKT cells has a unique mode of glycosphingolipid antigen recognition. Proc Natl Acad Sci USA 2004; 101: 12254-12259.
21. Godfrey DI, Hammond KJ, Poulton LD, Smyth MJ, Baxter AG. NKT cells: facts, functions and fallacies. Immunol Today 2000;21:573-583.
22. Berzins SP, Smyth MJ, Godfrey DI. Working with NKT cells - pitfalls and practicalities. Curr Opin Immunol 2005;17:448-454.
23. Van den Beemd R, Boor PP, Van Lochem EG, Hop WC, Langerak AW, Wolvers-Tettero ILM, Hooijkaas H, Van Dongen JJM. Flow cytometric analysis of the Vbeta repertoire in healthy controls. Cytometry 2000;40:336-345.
24. Verschuren MC, Wolvers-Tettero IL, Breit TM, Noordzij J, van Wering ER, van Dongen JJM. Preferential rearrangements of the T cell receptor-deltadeleting elements in human T cells. J Immunol 1997;158:1208-1216.
25. Breit TM, Verschuren MCM, Wolvers-Tettero ILM, Van Gastel-Mol EJ, Hählen K, Van Dongen JJM. Human T cell leukemias with continuous V(D)J recombinase activity for T cell receptor-δ gene deletion. J Immunol 1997;159:4341-4349.
26. Hazenberg MD, Otto SA, Cohen Stuart JW, Verschuren MCM, Borleffs JC, Boucher CA, Coutinho RA, Lange JM, Rinke de Wit TF, Tsegaye A, Van Dongen JJM, Hamann D, de Boer RJ, Miedema F. Increased cell division but not thymic dysfunction rapidly affects the T-cell receptor excision circle content of the naive T cell population in HIV-1 infection. Nat Med 2000;6:1036-1042.
27. Pongers-Willemse MJ, Verhagen OJHM, Tibbe GJM, Wijkhuijs JM, De Haas V, Roovers E, Van der Schoot CE, Van Dongen JJM. Real-time quantitative PCR for the detection of minimal residual disease in acute lymphoblastic leukemia using junctional regions specific TaqMan probes. Leukemia 1998;12:2006-2014.
28. Van der Velden VHJ, Hochhaus A, Cazzaniga G, Szczepanski T, Gabert J, Van Dongen JJM. Detection of minimal residual disease in hematologic malignancies by real-time quantitative PCR: principles, approaches, and laboratory aspects. Leukemia 2003;17:1013-1034.
29. Moreau EJ, Langerak AW, Van Gastel-Mol EJ, Wolvers-Tettero ILM, Zhan M, Zhou Q, Koop BF, Van Dongen JJM. Easy detection of all T cell receptor gamma (TCRG) gene rearrangements by Southern blot analysis: recommendations for optimal results. Leukemia 1999;13:1620-1626.
30. Szczepanski T, Langerak AW, Willemse MJ, Wolvers-Tettero ILM, Van Wering ER, Van Dongen JJM. T cell receptor gamma (TCRG) gene rearrangements in T cell acute lymphoblastic leukemia reflect 'end-stage' recombinations: implications for minimal residual disease monitoring. Leukemia 2000;14:1208-1214.
31. Van der Velden VHJ, Wijkhuijs JM, Jacobs DCH, Van Wering ER, Van Dongen JJM. T cell receptor gamma gene rearrangements as targets for detection of minimal residual disease in acute lymphoblastic leukemia by real-time quantitative PCR analysis. Leukemia 2002;16:1372-1380.
32. Gabert J, Beillard E, Van Der Velden VH, Bi W, Grimwade D, Pallisgaard N, Barbany G, Cazzaniga G, Cayuela JM, Cave H, Pane F, Aerts JL, De Micheli D, Thirion X, Pradel V, Gonzalez M, Viehmann S, Malec M, Saglio G, Van Dongen JJM. Standardization and quality control studies of "real-time" quantitative reverse transcriptase polymerase chain reaction (RQ-PCR) of fusion gene transcripts for minimal residual disease detection in leukemia - A Europe Against Cancer Program. Leukemia 2003;17:2318-2357.

## Claims

1. A method for determining the replicative history of a T-cell or a B-cell subset, comprising:
- detecting a signal joint nucleotide sequence on a TREC (T-cell receptor excision circle) or a BREC (B-cell receptor excision circle) in at least one T-cell or one B-cell of said subset; and
- detecting a coding joint nucleotide sequence that is formed upon deletion of said TREC or BREC from a chromosome during a TCR or Ig gene rearrangement in said T-cell or B-cell,
wherein said TCR or Ig gene rearrangement is the last single step V to J gene rearrangement before a functional TCR or Ig molecule is formed and wherein the chromosomal coding joint nucleotide sequence is stably retained in the chromosome.

2. A method according to claim 1, further comprising calculating the ratio between said coding joint nucleotide sequence and said chromosomal signal joint nucleotide sequence.

3. A method according to claim 1 or 2, wherein said TCR or Ig gene rearrangement is a *TCRG* (Vγ-Jγ) or a TCRA (Vα-Jα) rearrangement, or an *IGK* (Vκ-Jκ) or an *IGL* (Vλ-Jλ) rearrangement, respectively.

4. A method according to claim 3, wherein said T cell subset comprises antigen-selected TCRγδ⁺ T lymphocytes and wherein said TCR rearrangement is the Vγ9-Jγ1.2 rearrangement.

5. A method according to claim 3, wherein said T cell subset comprises NKT-cells and wherein said TCR rearrangement is the Vα10-Jα18 rearrangement.

6. A method according to any one of claims 1 to 5, wherein detecting involves polymerase chain reaction (PCR) analysis, preferably real-time quantitative (RQ) PCR analysis using SYBR Green I Dye, hydrolysis probes or hybridisation probes.

7. A nucleic acid amplification primer selected from those set out in Table 1 or 2, or a variant thereof.

8. A set of at least two pairs of nucleic acid amplification primers comprising at least a first pair of primers for detecting a signal joint nucleotide sequence on a TREC (T-cell receptor excision circle) or a BREC (B-cell receptor excision circle) and a second pair of primers for detecting a chromosomal coding joint nucleotide sequence in a T-cell subset or B-cell subset, wherein said TREC or BREC and said a coding joint nucleotide sequence are formed during the last single step V to J TCR or Ig gene rearrangement before a functional TCR or Ig molecule is formed and wherein the chromosomal coding joint nucleotide sequence is stably retained in the chromosome.

9. A set according to claim 8, wherein said TCR rearrangement is a *TCRG* or a *TCRA* rearrangement or wherein said Ig rearrangement is an *IGK* or an *IGL* rearrangement.

10. A set according to claims 8 or 9, wherein said TCR rearrangement is the Vγ9-Jγ1.2 rearrangement or the Vα10-Jα18 rearrangement.

11. A set according to any one of claims 8 to 10, wherein at least one of the primers is selected from those set out in Table 1 or Table 2, or a variant thereof.

12. A nucleic acid amplification assay, preferably PCR, more preferred RQ-PCR, using a set of primers according to any one of claims 8 to 10.

13. An oligonucleotide probe homologous to an internal sequence of an amplified nucleic acid sequence produced in a nucleic acid amplification assay according to claim 12.

14. An oligonucleotide probe selected from those set out in Table 1 or Table 2.

15. A T-cell or B-cell comprising a coding joint nucleotide sequence that is stably retained in the chromosome and that is the result of the last single step V to J TCR or Ig gene rearrangement before a functional TCR or Ig molecule is formed, said cell being stably transformed with one copy of a recombinant nucleic acid sequence that is homologous to the corresponding signal joint nucleotide sequence such that both the coding joint and signal joint sequence are replicated during cell division.

16. The T- or B-cell of claim 15, comprising in its genome the coding joint nucleic acid sequence and the corresponding signal joint nucleotide sequence formed during the Vγ9-Jγ1.2 or the Vα10-Jα18 rearrangement.

17. A diagnostic kit comprising a nucleic acid amplification primer of claim 7, a set of nucleic amplification primers according to any one of claims 8 to 11, and/or an oligonucleotide probe according to claim 13 or 14 and/or a cell according to claim 15 or 16.

18. Use of a method according to any one of claims 1 to 6 or a kit according to claim 17 to determine the replicative history of a normal or a diseased T-cell or B-cell subset, preferably to assess normal and aberrant T-cell or B-cell expansion.
